(19) Europäisches Patentamt
European Patent Office
Office européen des brevets

(11) **EP 4 160 611 A1**

(12) **EUROPEAN PATENT APPLICATION**

(43) Date of publication:
**05.04.2023 Bulletin 2023/14**

(51) International Patent Classification (IPC):
***G16H 40/20*** (2018.01)

(21) Application number: **22198884.3**

(22) Date of filing: **29.09.2022**

(52) Cooperative Patent Classification (CPC):
**G16H 40/20; G16H 50/70**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2021 JP 2021160732**

(71) Applicant: **Canon Medical Systems Corporation Tochigi 324-0036 (JP)**

(72) Inventors:
• **KANO, Yusuke**
**Tochigi, 324-0036 (JP)**
• **SASAKI, Sho**
**Tochigi, 324-0036 (JP)**

(74) Representative: **Marks & Clerk LLP**
**15 Fetter Lane**
**London EC4A 1BW (GB)**

(54) **MEDICAL INFORMATION PROCESSING APPARATUS, MEDICAL INFORMATION PROCESSING METHOD, AND RECORDING MEDIUM**

(57) A medical information processing apparatus according to an embodiment includes processing circuitry. The processing circuitry acquires an effect evaluation value regarding a plurality of medical decisions. The processing circuitry acquires a fairness index value regarding the aforementioned medical decisions. The processing circuitry determines a medical decision to be assigned to a subject target based on the effect evaluation value and the fairness index value.

F I G. 1

EP 4 160 611 A1

**Description**

CROSS-REFERENCE TO RELATED APPLICATIONS

This application is based upon and claims the benefit of priority from Japanese Patent Application No. 2021-160732, filed September 30, 2021, the entire contents of which are incorporated herein by reference.

FIELD

Embodiments described herein relate generally to a medical information processing apparatus, a medical information processing method, and a recording medium.

BACKGROUND

Assigning a given medical decision from a number of medical decisions to a patient is practiced in clinical researches and daily medical treatment. Adaptive design, bandit algorithms, etc. are known techniques to adaptively tailor such assignments. In order to maximum benefits for patients in general, a medical decision which is less likely to be best needs be selected deliberately at an appropriate frequency. However, this causes unfairness between patients because an estimate value of a magnitude of an effect by each clinical medical decision is clear when it is assigned.

SUMMARY

In relation to the embodiments, the following matters are disclosed as one aspect and a selective feature of the present invention.

1. A medical information processing apparatus comprising processing circuitry configured to:

acquire an effect evaluation value regarding a plurality of medical decisions;
acquire a fairness index value regarding the medical decisions; and
determine a medical decision to be assigned to a subject target based on the effect evaluation value and the fairness index value.

2. The fairness index value may be a value based on the effect evaluation value. The processing circuitry may calculate the fairness index value based on the effect evaluation value.
3. The fairness index value is a value based on the effect evaluation value and a target value with respect to the effect evaluation value set for a respective one of subjects including the subject target. The processing circuitry may be configured to calculate the fairness index value based on the effect evaluation value and the target value.
4. The processing circuitry may set the target value based on an effect evaluation value regarding a past medical decision for the subject target.
5. The processing circuitry may be configured to set the target value based on an effect evaluation value regarding a medical decision selected by a subject other than the target subject.
6. The processing circuitry may be configured to:

determine an assignment candidate from among the plurality of medical decisions based on the effect evaluation value for a respective one of the medical decisions; and
determine the medical decision to be assigned based on the fairness index value of the assignment candidate.

7. Wherein in a case where a fairness index value regarding the assignment candidate satisfies a predetermined condition, the processing circuitry may determine that the assignment candidate is the medical decision to be assigned.
8. Wherein the processing circuitry may be configured to:

stochastically sample a plurality of assignment candidates from the plurality of medical decisions based on a plurality of random numbers in accordance with a predetermined policy; and
determine the medical decision to be assigned based on the fairness index value of the plurality of assignment candidates.

9. Wherein the processing circuitry may determine the medical decision to be assigned based on a comparison of fairness index values between the assignment candidates.

10. Wherein the effect evaluation value is based on a posterior distribution of an effect with respect to a respective one of the medical decisions.

11. Wherein the effect evaluation value is calculated based on an expression:

$$p_i = \int_{-\infty}^{\infty} q_i(\alpha|D_i) \prod_{i \neq j} \left( \int_{-\infty}^{\alpha} q_j(\beta|D_j) \, d\beta \right) d\alpha$$

$q_i(\alpha|D_i)$ is a first posterior probability density function of an effect of medical decision i under a condition in which effect observation value $D_i$ is obtained, and $\alpha$ is a parameter of the first posterior probability density function. $q_j(\beta|D_j)$ is a second posterior probability density function of an effect of medical decision j under a condition in which effect observation value $D_j$ is obtained, and $\beta$ is a parameter of the second posterior probability density function.

12. A medical information processing method comprising:

acquiring an effect evaluation value regarding a plurality of medical decisions;
acquiring a fairness index value regarding the medical decisions; and
determining a medical decision to be assigned to a subject target based on the effect evaluation value and the fairness index value.

13. A non-transitory computer-readable storage media recording a medical information processing program for causing a computer to implement functions of:

acquiring an effect evaluation value regarding a plurality of medical decisions;
acquiring a fairness index value regarding the medical decisions; and
determining a medical decision to be assigned to a subject target based on the effect evaluation value and the fairness index value.

BRIEF DESCRIPTION OF THE DRAWINGS

FIG. 1 is a diagram showing an exemplary configuration of a medical information processing apparatus according to an embodiment.
FIG. 2 is a flowchart showing medical information processing performed by processing circuitry in accordance with a medical information processing program.
FIG. 3 is a diagram schematically illustrating the medical information processing shown in FIG. 2.
FIG. 4 is a flowchart showing assignment processing performed by the processing circuitry according to Example 1.
FIG. 5 is a diagram schematically illustrating the medical information processing shown in FIG. 4.
FIG. 6 is a diagram showing a concept of a subjective best arm probability and a subjective best arm probability difference.
FIG. 7 is a flowchart showing a fairness index value in the case of a target value having a range.
FIG. 8 is a flowchart showing assignment processing performed by processing circuitry according to Example 2.
FIG. 9 is a diagram schematically illustrating the medical information processing shown in FIG. 8.
FIG. 10 is a diagram showing a fairness index value in the case of a target value being a single value.
FIG. 11 is a diagram showing changes in assignment policy for medical decisions with a single target value.
FIG. 12 is a diagram showing a specific example of setting processing of an assignment subject.
FIG. 13 is a diagram showing another specific example of setting processing of an assignment subject.

DETAILED DESCRIPTION

A medical information processing apparatus according to an embodiment includes processing circuitry. The processing circuitry acquires an effect evaluation value regarding a number of medical decisions. The processing circuitry acquires a fairness index value regarding the aforementioned medical decisions. The processing circuitry determines a medical decision to be assigned to a subject target based on the effect evaluation value and the fairness index value.

Embodiments of a medical information processing apparatus, a medical information processing method, and a recording medium will be described in detail with reference to the drawings.

In the medical field, efforts to realize clinical decision support (CDS) using technologies based on data and artificial intelligence (AI) are ongoing. AI technologies employ supervised learning as represented by deep learning, and some of these techniques are beginning to have a lesion detecting capacity outrunning, in especially the image diagnosis domain, the accuracy of a human being.

However, the following circumstances are expected to hinder the realization of clinical decision support (CDS) through supervised learning. First, improvement in accuracy requires an enormous amount of data, which would incur large costs for data collection. Second, despite the related high accuracy, a proper clinical decision may not result.

The second issue is technically synonymous with a problem that supervised learning, while being capable of revealing a correlation, cannot demonstrate a causal sequence. For example, even if an AI model can predict the within-5-year death of a heart failure patient with 100% accuracy, such an AI model cannot assist a doctor who wants to know the method for extending the remaining life of this patient to 5 years or longer.

Demonstrating a causal sequence in medical activities to thereby specify the best medical option is yet to be realized by the supervised learning of the day, and this is a role of randomized controlled trials (RCT) or medical statistics. In other words, as long as the current AI technologies prevail, a clinical decision support (CDS) for the optimized treatment cannot be realized in the true sense, and randomized controlled trials (RCT) will not disappear.

Randomized controlled trials (RCT) also involve many problems. For example, these problems include, first, the significant amount of time required to complete the research, which makes it impossible to follow the latest trends, thus the resultant findings could easily become obsolete. Second, the research is very costly, which is also accompanied by a risk of failure to prove the hypothesis, thus efficiency is not guaranteed. Third, in general, eligibility criteria set for the research are strict, which could render the resultant findings inapplicable for ordinary patients (i.e., result in low external validity). Fourth, as a control, only an average intervention effect can be employed, which does not allow for effects that vary among individual patients to be specified. For example, cases such as personalized medicine cannot be assumed. Fifth, statistical significance could be overly relied upon, which may easily lead to publication bias, p-hacking, etc.

In particular, currently, treatment methodologies are frequently updated and renewed, which together raise the importance of personalized medicine. Thus, the limits of randomized controlled trials (RCT) are becoming apparent.

Accordingly, there is a demand for efficient optimization of medical decisions that does not require a large amount of data in advance and that can be performed without randomized controlled trials (RCT). Furthermore, for this purpose, formulation of models for clinical decision support (CDS) is desired.

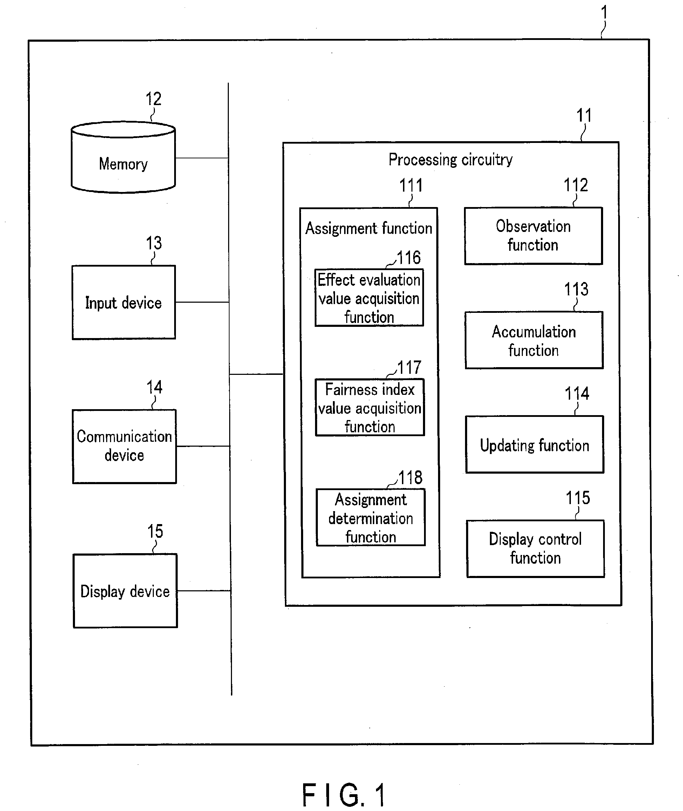

FIG. 1 is a diagram showing an exemplary configuration of a medical information processing apparatus 1 according to an embodiment. As shown in FIG. 1, the medical information processing apparatus 1 is an information processing terminal which may be a computer, etc., including processing circuitry 11, a memory 12, an input device 13, a communication device 14, and a display device 15. The processing circuitry 11, the memory 12, the input device 13, the communication device 14, and the display device 15 are connected with one another via a bus so that they can mutually input and output signals.

The processing circuitry 11 includes one or more processors such as a central processing unit (CPU), a graphics processing unit (GPU), and so on. The processing circuitry 11, by running one or more medical information processing programs, implements various functions, including an assignment function 111, an observation function 112, an accumulation function 113, an updating function 114, and a display control function 115. Note that the functions 111 to 115 are not limited to the implementation through a single processing circuitry component. Multiple independent processors may be employed together to form processing circuitry so that the respective processors run the programs to realize the functions 111 to 115. Moreover, the functions 111 to 115 may be respective programs serving as modules to constitute a medical information processing program. Such programs may be stored in the memory 12.

The memory 12 is a storage device adapted to store various types of information sets, such as one or any combination of a read only memory (ROM), a random access memory (RAM), a hard disk drive (HDD), a solid state drive (SSD), an integrated circuit memory device, and so on. Other than such storage devices, the memory 12 may be one or any combination of non-transitory computer-readable storage media such as a compact disc (CD), a digital versatile disc (DVD), and a flash memory, or a driver that reads and writes various types of information sets with semiconductor memory devices. Also, the memory 12 may be located within an external computer connected via a network.

The input device 13 receives various input operations from an operator and converts the received input operations into electrical signals for output to the processing circuitry 11. More specifically, the input device 13 is coupled to one or more input instruments such as a mouse, a keyboard, a track ball, switches, buttons, a joystick, a touch pad, and a touch panel display. The input device 13 outputs electric signals corresponding to input operations received by such input instruments to the processing circuitry 11. Note that the input device 13 may be furnished at an external computer connected via a network, etc.

The communication device 14 is an interface for exchanging various types of information sets with one or more external computers. The communication device 14 performs data communication according to the standard suitable for medical information communication, such as a digital imaging and communications in medicine (DICOM).

The display device 15 displays various types of information sets according to the display control function 115 of the processing circuitry 11. The display device 15, for example, may be a liquid crystal display (LCD), a cathode ray tube (CRT) display, an organic electroluminescence display (OELD), a plasma display, or any other display available. The display device 15 may instead be a projector.

The processing circuitry 11 according to the embodiment, with the assignment function 111, determines a medical decision to be assigned to a subject such as a patient, based on an effect evaluation value for the medical decision. Such effect evaluation values for medical decisions are calculated using models adapted to calculate the effect evaluation values for medical decisions. The subject receives a medical act corresponding to the assigned medical decision. An effect attributable to the medical act is produced for the subject. The processing circuitry 11, with the observation function 112, observes the effect produced for the subject. The effect is represented by a numerical value. This numerical value will be called an "effect observation value". With the accumulation function 113, the processing circuitry 11 accumulates observation data, in which the effect observation value, the medical act, and other feature amount or amounts are associated with each other, in the memory 12. Such assignment of a medical decision, observation of an effect, and accumulation of observation data are conducted for various subjects. By implementing the updating function 114, the processing circuitry 11 updates, during the accumulation of observation data, models based on the observation data. By implementing the display control function 115, the processing circuitry 11 presents various data sets through the display device 15.

The assignment function 111 is divided into an effect evaluation value acquisition function 116, a fairness index value acquisition function 117, and an assignment determination function 118. By implementing the effect evaluation value acquisition function 116, the processing circuitry 11 acquires an effect evaluation value regarding a plurality of medical decisions. By implementing the fairness index value acquisition function 117, the processing circuity 11 acquires a fairness index value regarding a plurality of medical decisions. By implementing the assignment determination function 118, the processing circuitry 11 determines a medical decision to be assigned to a subject target based on an effect evaluation value and a fairness index value.

The subject in the context of the present embodiment is typically a single patient suffering from a disease. However, the subject assumed by the embodiment is not limited to this but may be a patient group constituted by two or more patients. Also, the subject in the embodiment is not limited to a person suffering from a disease, but may be a healthy person. The models may be employed for respective doctors, respective treatment departments, respective hospitals, or respective geographical areas. The models may also be employed for respective subjects.

Each model according to the embodiment is a function for calculating the effect evaluation value for a medical decision. This function may be a function defined by a manual operation, a function defined by an experimental approach or a deterministic approach, a function formed through training in machine learning, or a function defined by any other method.

The embodiment assumes types of medical decisions including execution/non-execution-related decisions, content-related decisions, amount-related decisions, and time-related decisions. Examples of the execution/non-execution-related decisions include a decision as to whether or not to execute a surgery or whether or not to execute a blood test. Examples of the content-related decisions include a decision for the selection of a drug to be used or the selection of a disease name based on which diagnosis is conducted. Examples of the amount-related decisions include a decision for the selection of a dose of pharmaceuticals or the selection of a rehabilitation duration. Examples of the time-related decisions include a decision for the selection of a time to visit a hospital or the selection of a time to execute a surgery.

Persons who make medical decisions are not limited to medical professionals such as doctors, nurses, and other health-care providers, but may include subjects themselves, family members of subjects, and any other persons associated with subjects. Medical decisions are not always required to be advanced or sophisticated decisions for healthcare, nor are they limited to the purposes of health improvement. That is, medical decisions may cover decisions that lead to a worse result for medicine, health, etc. For example, a decision as to whether or not a healthy individual smokes a cigarette at a given timing is also assumed to be a medical decision.

A medical decision consequently results in a certain effect. Such an effect is called a "reward" or an "outcome". Effects discussed in the context of the embodiment are assumed to be, for example, clinical outcomes, patient-reported outcomes, and economic outcomes. Examples of the clinical outcomes include a morbidity rate (including whether or not a subject suffers from a disease or the like), a 5-year survival rate (including whether or not a subject is alive), a complication incidence rate (including whether or not a subject suffers from complications), a re-hospitalization rate (including whether or not a subject is hospitalized again), test values or an improvement degree of test values, and an independence degree in daily life. Examples of the patient-reported outcomes include a subjective symptom, a subjective health condition, a satisfaction level of treatment, and a subjective level of happiness. Examples of the economic outcomes include medical costs, invested medical resources, and a length of stay at a hospital.

The effects may be given as numerical representations, to which a superiority-and-inferiority assessment can be done for learning. Numerical values may be assigned to items that are not originally numerical representations. The effects may include those readily observable after a medical decision. For example, an effect of a medical decision as to whether or not to send a message prompting an exercise to the smartphone of a healthy individual may be a fact of conducting or not conducting an exercise within 5 minutes of the receipt of the message.

The effects may take into consideration the costs required for a medical decision. For example, in the instances of a smartphone message as discussed, sending a message would provide merits of more easily obtaining the effects than not sending a message, but it could concurrently cause demerits of incurring a communication cost, restricting the user activities, etc. One exemplary implementation for taking such merits and demerits into consideration may be to assume, for example, that sending a first message always incurs a cost of "5" and conducting an exercise gives a reward of "100", and calculating the value of the effect as 100 (reward) - 5 (cost) = 95. In this manner, effects reflecting cost effectiveness can be obtained.

Feature amounts according to the embodiment include one or more attributes and/or one or more conditions of a subject. The attributes here are information items which, by nature, are not changed according to a medical decision just performed, and they include the sex, age, etc. of a subject. The conditions here are information items which, by nature, are changed according to a medical decision just performed, and they include a current blood pressure, a current blood glucose level, etc. of a subject.

Hereinafter, the medical information processing apparatus 1 according to the present embodiment is described.

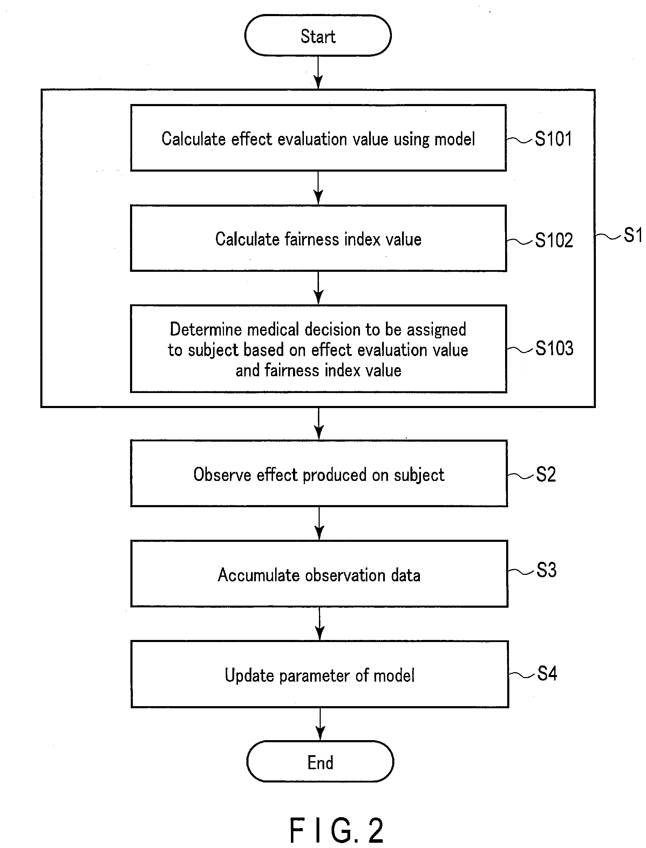

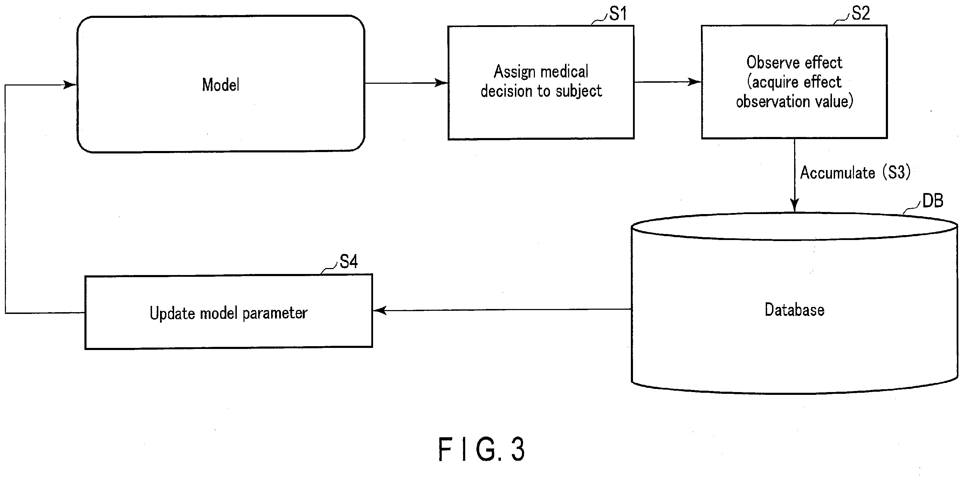

FIG. 2 is a flowchart showing medical information processing 11 that is conducted by processing circuitry in accordance with a medical information processing program. FIG. 3 is a diagram schematically illustrating the medical information processing shown in FIG. 2.

As shown in FIGS. 2 and 3, by implementing the assignment function 111, the processing circuitry 11 assigns a medical decision to a subject entity using a model therefor (step S1). In step S1, the processing circuitry 11 determines a medical decision for an assignment subject in accordance with an algorithm based on a bandit algorithm. The bandit algorithm is an algorithm that aims to optimize the trade-off between exploration and utilization, and sequentially determines medical decisions in such a manner as to maximize a cumulative remuneration to be acquired or to minimize a cumulative regret (herein, a regret represents a difference between a remuneration acquired from the optimal behavior and a remuneration acquired from the actual behavior). Even with the cumulative remuneration being maximum or the cumulative regret being minimum, the fairness of exploration is not necessarily ensured. Accordingly, the processing circuitry 11 determines a medical decision serving as an assignment subject, based on an effect evaluation value and a fairness index value in order to realize the maximized accumulative remuneration or the minimized accumulative regret while maintaining the fairness of exploration. More specifically, in step S1, assignment of a medical decision is performed by the following process.

The bandit algorithms in the embodiment include not only narrowly-defined bandit algorithms where effects do not depend on a feature amount, but also contextual bandit algorithms where effects depend on a feature amount. The bandit algorithms in the embodiment further include reinforcement learning for solving a sequential decision-making problem where conditions vary according to the medical decisions so far. Examples of the concrete algorithms that may be used as the bandit algorithms in the embodiment include epsilon greedy, Thompson sampling, linear Thompson sampling, posterior sampling for reinforcement learning (PSRL), and Bayesian deep Q-networks (BDQN).

First, by implementing the effect evaluation value acquisition function 116, the processing circuitry 11 calculates a plurality of effect evaluation values respectively corresponding to a plurality of medical decisions by using a model (Step S101). The effect evaluation value is an index value for evaluating the effect of an assignment candidate medical decision, and examples of such an index value include a subjective best arm probability, a subjective best arm probability difference, a confidence interval of the effect, an expected value of the effect, a maximum value of this expected value, a difference from this maximum value of the expected value, and so on.

After step S101, by implementing the fairness index value acquisition function 117, the processing circuitry 11 calculates a plurality of fairness index values respectively corresponding to a plurality of medical decisions (step S102). The fairness index value is calculated for each medical decision. The fairness index value is an index value for evaluating the degree of fairness in selecting a corresponding medical decision. The fairness index value is an index value based on an effect evaluation value. More specifically, the fairness index value is calculated based on the effect evaluation value and a target value for the fairness index value. The target value for the fairness index value according to the present embodiment may be either a value having a range or a single value.

After step S102, by implementing the assignment determination function 118, the processing circuitry 11 determines a medical decision to be assigned to a subject, based on the effect evaluation value calculated in step S101 and the fairness index value calculated in step S102 (S103). Specifically, the processing circuitry 11 according to Example 1 below determines a single random number according to a predetermined probability distribution, and determines, based on the determined single random number, one or more assignment candidates by selecting it or them from among a plurality of medical decisions according to a predetermined policy. In the case where the fairness index value regarding an assignment candidate satisfies a predetermined condition, the processing circuitry 11 determines that this assignment candidate is a medical decision to be assigned as an assignment subject. The processing circuitry 11 according to Example 2 below determines a plurality of random numbers in accordance with a predetermined probability distribution by selecting them from among a plurality of medical decisions, and based on the determined random numbers, stochastically determines a plurality of assignment candidates in accordance with a predetermined policy. The processing circuitry 11 determines a medical decision to be assigned an assignment subject, based on a comparison of fairness index values between the assignment candidates.

After step S103, a medical act corresponding to the medical decision is performed on a subject. At this time, a medical act corresponding to a medical decision selected by the subject himself/herself, not a medical decision determined in step S103, may be performed.

After step S1, the processing circuitry 11 with the observation function 112 observes the effect produced for the subject (step S2). The effect is observed in the form of numerical value or values, i.e., effect observation values. For example, if the medical decision indicates "Execute a surgery", information such as a 5-year survival rate, the presence of complication incidence, and so on are acquired as the effect observation values. The effect observation values may be acquired by any method. For example, an operator may input them via the input device 13, or a testing instrument may input its measurement values. The effect observation values may be received from one or more external computers via the communication device 14.

After step S2, by implementing the accumulation function 113, the processing circuitry 11 accumulates the observation data in a database DB managed under the memory 12 (step S3). The observation data includes an identifier of the subject, the medical decision corresponding to the medical act performed on the subject, and the effect observation value or values from the effect produced for the subject by the medical act.

After step S3, the processing circuitry 11 updates a model by implementing the updating function 114 (step S4). As an example, the processing circuitry 11 updates parameters of the model each time an effect is observed, in other words, each time an effect observation value is acquired.

The medical information processing according to the present embodiment is thus completed. Steps S1 to S4 are then repeatedly performed on the same subject or different subjects. This improves the calculation accuracy of the effect evaluation value of the model and also improves the allocation accuracy of medical decisions.

Several examples of the present embodiment will be described below.

Example 1

The bandit algorithm such as Thompson sampling is a method that theoretically guarantees that the number of options under a maximum value of an expected value is drawn the "minimum necessary" number of times; however, this "minimum necessary" indicates the selection of all options including an infinite number of subjects, and under the presence of a finite number of subjects, unnecessary explorations are considered to exist. For example, the effect obtained by selecting an option that is far less likely to have a maximum value of an expected value for a subject will occur far in the future after the present time. On the other hand, such an exploration result in selection of an option that is far less likely to have a maximum value of an expected value, thereby significantly reducing fairness between subjects. As described above, in the case of assuming the limited number of times, there is a probability that both of the regret and the fairness will be improved by deleting some of the explorations.

The processing circuitry 11 according to Example 1 sets a threshold value for an effect evaluation value, and executes a method (clipping) of avoiding selection of a medical decision having the effect evaluation value greater than the aforementioned threshold value. That is, in Example 1, a target value for the effect evaluation value is set to a value having a range. The medical information processing according to Example 1 will be described below. Suppose the effect evaluation value according to Example 1 corresponds to the subjective best arm probability and/or the subjective best arm probability difference.

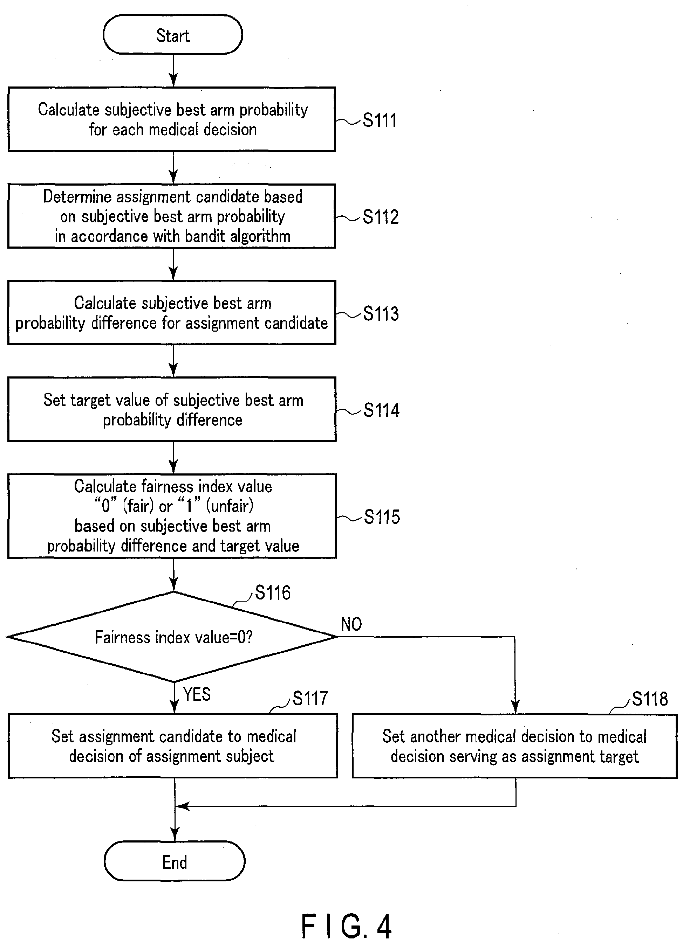

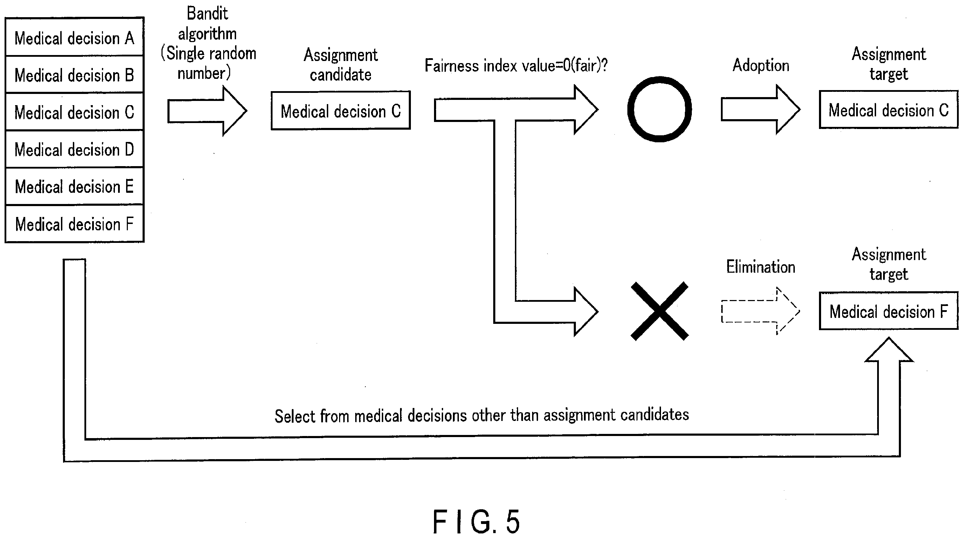

FIG. 4 is a flowchart showing the assignment processing performed by the processing circuitry 11 according to Example 1. FIG. 5 is a diagram schematically illustrating the medical information processing shown in FIG. 4. Steps S111 to S118 shown in FIG. 4 are included in step S1 shown in FIG. 2. Suppose the medical information processing shown in FIG. 4 is the assignment step (S1) repeatedly performed in FIG. 2, in particular, the assignment step performed for the n-th time. In other words, it is assumed that assignment of a medical decision has already been performed multiple times for the same subject or different subjects.

First, by implementing the effect evaluation value acquisition function 116, the processing circuitry 11 calculates the subjective best arm probability for each medical decision (step S111). The subjective best arm probability is a subjective probability of an event that, at any given time, an expected value of an effect of the corresponding medical decision reaches its maximum value. The subjective probability means the degree of subjective belief or certainty based on information and experience gained by an individual and algorithm. The subjective best arm probability is calculated based on the posterior distribution of an effect with respect to each medical decision based on Bayes' theorem in accordance with the following expression (1). Herein, $q_i(\alpha|D_i)$ is the posterior probability density function of an effect of medical decision i under the condition in which observation data (effect observation value) $D_i$ is obtained, and $\alpha$ is a

parameter of the probability density function. Furthermore, $q_i(\beta|D_j)$ is the posterior probability density function of an effect of medical decision j under the condition in which observation data (effect observation value) $D_j$ is obtained, and $\beta$ is a parameter of the probability density function. The subjective best arm probability can be calculated by Monte Carlo simulation, etc.

$$p_i = \int_{-\infty}^{\infty} q_i(\alpha|D_i) \prod_{i \neq j}\left(\int_{-\infty}^{\alpha} q_j\left(\beta|D_j\right) d\beta\right) d\alpha \qquad (1)$$

After step S111, by implementing the assignment determination function 118, the processing circuitry 11 determines an assignment candidate based on the subjective best arm probability according to the bandit algorithm (step S112). A specific policy algorithm of the bandit algorithm is not particularly limited and may employ an Epsilon Greedy method, an Upper Confidence Bound (UCB) method, a Minimum Empirical Divergence (MED) method, a Thompson sampling, etc. For example, in the case of using Thompson sampling, the processing circuitry 11 probabilistically determines a clinical decision in such a manner that the assignment probability (sampling probability) of each medical decision matches the subjective best arm probability. The determined medical decision is set to an assignment candidate.

As shown in FIG. 5, in Example 1, it is assumed that a medical decision to be assigned as an assignment subject is determined by selecting it from six medical decisions A to F. At step S111, the processing circuitry 11 first samples a single random number from a given probability distribution. The processing circuitry 11 then determines by selecting one assignment candidate from among the six medical decisions A to F according to a predetermined policy algorithm based on a single random number. The predetermined policy algorithm is not limited to a probabilistic policy, but may be a deterministic policy and is freely selectable from the above policy algorithms. FIG. 5 assumes that a medical decision C is determined to be an assignment candidate.

After step S112, by implementing the effect evaluation value acquisition function 116, the processing circuitry 11 calculates the subjective best arm probability difference for the assignment candidate (step S113). The subjective best arm probability is an example of an effect evaluation value. The subjective best arm probability difference $\Delta p_i$ is a value obtained by subtracting the subjective arm probability $p_j$ of a medical decision as a calculation subject from the maximum value $\max_j p_j$ of the subjective best arm probability of all medical decisions, as expressed in the following expression (2).

$$\Delta p_i = \max_j p_j - p_i \qquad (2)$$

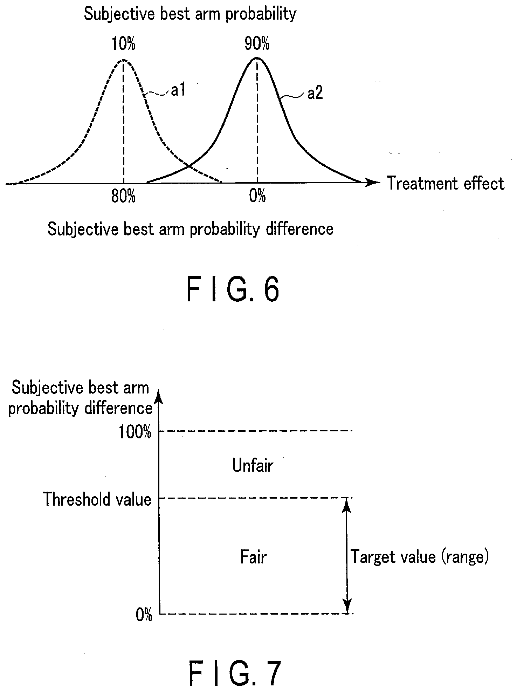

FIG. 6 is a diagram showing a concept of the subjective best arm probability and a subjective best arm probability difference. As shown in FIG. 6, a posterior distribution a1 of a treatment effect with respect to a medical decision 1 and a posterior distribution a2 of a treatment effect with respect to a medical decision 2 are illustrated. The treatment effect is an example of the effect of medical decision. Given that the subjective best arm probability of the medical decision 1 is equal to 10% and the subjective best arm probability of the medical decision 2 is equal to 90%, the maximum value of the subjective best arm probability between the medical decisions 1 and 2 is equal to 90%. Thus, the subjective best arm probability difference of the medical decision 1 is expressed as 90%-10%=80% and the subjective best arm probability difference of the medical decision 2 is expressed as 90%-90%=0%.

After step S113, by implementing the fairness index value acquisition function 117, the processing circuitry 11 sets a target value of the subjective best arm probability difference (step S114). The target value according to Example 1 is set within the range of the subjective best arm probability difference. After step S114, by implementing the fairness index value acquisition function 117, the processing circuitry 11 calculates a fairness index value based on the subjective best arm probability difference and the target value (step S115).

FIG. 7 is a flowchart showing the fairness index value in the case of the target value having a range. As shown in FIG. 7, a threshold value is set to a given value of the subjective best arm probability difference which corresponds to the effect evaluation value. A range equal to or smaller than the threshold of the subjective best arm probability difference is set to a target value. The fairness index value of "1" is assigned to the range from the threshold to the subjective best arm probability difference of 100%. The fairness index value of "1" means unfairness. The fairness index value of "0" is assigned to the range from the threshold to the subjective best arm probability difference of 0%, that is, a range of the target value. A fairness index value of "0" means fairness.

For example, in the case where the subjective best arm probability difference of the medical decision A is equal to 0% and the subjective best arm probability difference of the medical decision B is equal to 80%, the target value is fixed in the range from 0% to 40%. In this case, the subjective best arm probability difference of medical decision A falls within the range of the target value, so that the fairness index value is set to "0", indicating fairness. On the other hand, the subjective best arm probability difference of medical decision B falls outside the range of the target value, so that the fairness index value is set to "1", indicating unfairness.

The target value may be or may not be a fixed value over a plurality of processing target times (trials). Also, the target value may vary according to the number of trials. For example, the target value may be set such that it is narrowed, that is, the range of fairness is narrowed as the number of trials increases.

After step S115, by implementing the assignment determination function 118, the processing circuitry 11 determines whether or not the fairness index value of "0" is set to an assignment candidate in step S115 (step S116). If it is determined in step S116 that the fairness index value of "0" is set to an assignment candidate (step S116: YES), the processing circuitry 11 sets the assignment candidate to a medical decision to be assigned as an assignment subject (step S117). If it is not determined in step S116 that the fairness index value of "0" is set to an assignment candidate (step S116: NO), that is, if the fairness index value of "1" is set to the assignment candidate, the processing circuitry 11 sets another medical decision to a medical decision to be assigned as an assignment subject (step S118).

For example, as shown in FIG. 5, it is determined in step S116 whether or not the fairness index value of "0" has been set to the medical decision C which is an assignment candidate. If it is determined that the fairness index value of "0" has been set to the medical decision C, the medical decision C is adopted as an assignment subject. On the other hand, if it is determined that the fairness index value of "1" has been set to the medical decision C, the medical decision C is eliminated. In this case, the processing circuitry 11 may set, to an assignment subject, one of the medical decisions A, B, D, E, and F, which has the highest subjective best arm probability and is other than the assignment candidate. As another example, the processing circuitry 11 may probabilistically set an assignment subject by selecting it from some of the medical decisions A, B, D, E, and F, which have the fairness index value of "0" and are other than the assignment candidate. In FIG. 5, a medical decision F is set to an assignment subject.

The assignment processing according to Example 1 is thus completed.

As described above, the processing circuitry 11 according to Example 1 determines a single random number according to a predetermined probability distribution, and determines, based on the determined single random number, one or more assignment candidates by selecting it or them from among a plurality of medical decisions according to a predetermined policy. In the case where the fairness index value regarding an assignment candidate satisfies a predetermined condition, the processing circuitry 11 determines that this assignment subject is a medical decision to be assigned as an assignment subject. More specifically, in the case of the fairness index value of an assignment candidate being the predetermined value of "0", the processing circuitry 11 sets this assignment candidate to an assignment subject, and in the case of the fairness index value of an assignment candidate being not the predetermined value of "0", the processing circuitry 11 sets an assignment subject by selecting it from among medical decisions other than this assignment candidate. The method according to Example 1 is considered as modifying, based on the fairness index value, the assignment selected according to the bandit algorithm. The method according to Example 1 eliminates an assignment candidate lacking fairness from an assignment subject while setting a base to the bandit algorithm that selects a medical decision at an appropriate exploratory frequency, so that exploration can be conducted while ensuring fairness.

The method according to Example 1 allows the fairness index value to affect only a portion that selects a medical decision, thereby being applicable to any decision making form, such as context-free bandit, contextual bandit, and sequential decision making. The context-free bandit is such that an effect depends only on a medical decision. In the case where each subject selects one of the medical decisions of multiple types, an expected value of an effect on the subject is affected only by the selected type of medical decision. Such a case is generally referred to as multi-armed bandit. The contextual bandit is such that an effect depends not only on a medical decision but also on a feature amount of a patient. This is a case in which the same medical decision may have different effects depending on the subject who receives the medical decision. In such cases, optimizing a medical decision for each individual is equivalent to realizing precision medicine. The sequential decision making is such that selection of each subject is performed multiple times and each selection is not independent. For example, assume that the given amount of insulin (hormonal drug that lowers a blood sugar level) is regularly administered to a diabetic patient for blood sugar level control. In this case, the blood sugar level obtained as a result of this time's insulin administration depends on the blood sugar level before the administration, and this blood sugar level before the administration depends on the result of the previous insulin administration. As described above, the sequential decision making repeats operation in which the next decision making is performed based on an effect and a new feature amount observed as a result of one decision making. In medical care, the sequential decision making is equivalent to dynamically optimizing a clinical pathway (that defines the sequence of a series of treatments or examinations, and an implementation standard) and a decision on chronic stage management.

Example 2

The method based on Thompson sampling stochastically determines a medical decision. Thus, even in a presence of a preset target value of the subjective best arm probability difference, it is essentially impossible to approach this target value. Example 2 aims to approach a target value of the subjective best arm probability difference by allowing a certain flexibility in the selection of a medical decision.

In the sequential decision making, the processing circuitry 11 according to Example 2 executes a technique (targeting) using the target value setting in combination with the action setting method described above. Example 2 sets a single value to a target value for the subjective best arm probability difference. The medical information processing according to Example 2 will be described below. As in Example 1, assume that the effect evaluation value according to Example 2 is also the subjective best arm probability and/or the subjective best arm probability difference.

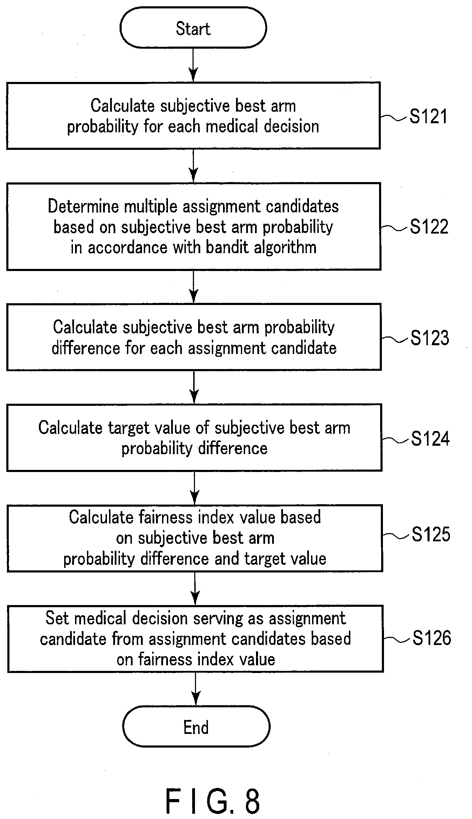

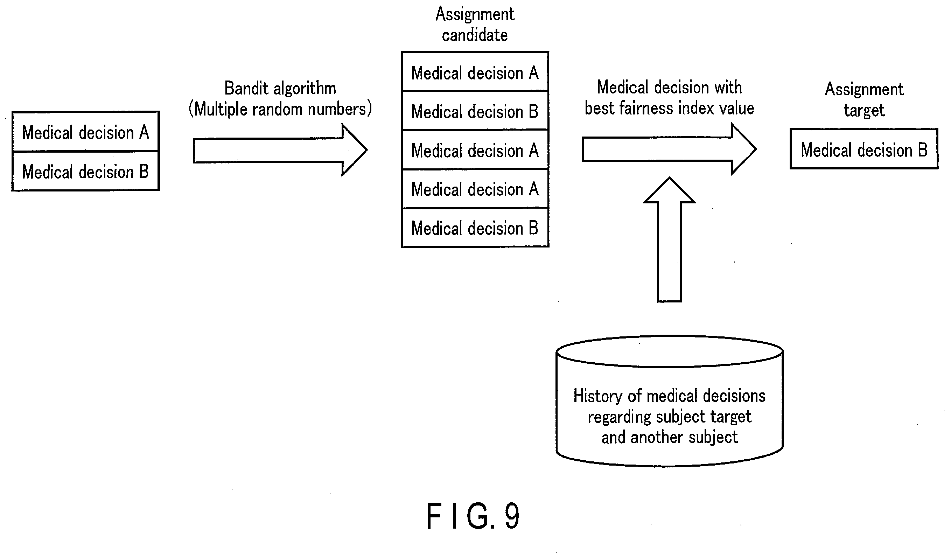

FIG. 8 is a flowchart showing assignment processing performed by the processing circuitry 11 according to Example 2. FIG. 9 is a diagram schematically illustrating the medical information processing shown in FIG. 8. Steps S121 to S126 shown in FIG. 8 are included in step S1 shown in FIG. 2. Suppose the medical information processing shown in FIG. 8 is the assignment step (S1) repeatedly performed in FIG. 2, in particular, the assignment step performed the n-th time. In other words, it is assumed that assignment of a medical decision has already been performed multiple times for the same subject or different subjects.

First, by implementing the effect evaluation value acquisition function 116, the processing circuitry 11 calculates the subjective best arm probability for each medical decision (step S121). Step S121 is similar to step Sill.

After step S121, by implementing the assignment determination function 118, the processing circuitry 11 determines a plurality of assignment candidates based on the subjective best arm probability according to the bandit algorithm (step S122). At step S122, first, the processing circuitry 11 independently samples a plurality of random numbers from a discretionary distribution. For example, as shown in FIG. 9, assume that an assignment subject is determined by selecting it from a medical decision A and a medical decision B. First, a plurality of (five in FIG. 9) random numbers are sampled from a discretionary probability distribution. The processing circuitry 11 then determines an assignment candidate by selecting it from the medical decision A and the medical decision B according to a predetermined algorithm based on each of the five random numbers. The predetermined algorithm is not limited to a probabilistic policy, but may be a deterministic policy and is arbitrarily selectable from the policy algorithms described above.

After step S122, by implementing the effect evaluation value acquisition function 116, the processing circuitry 11 calculates the subjective best arm probability difference for each assignment candidate (step S123). The method of calculating the subjective best arm probability difference is the same as in Example 1.

After step S123, by implementing the fairness index value acquisition function 117, the processing circuitry 11 sets a target value of the subjective best arm probability difference (step S124). The target value according to Example 2 is set to a single value. After step S124, by implementing the fairness index value acquisition function 117, the processing circuitry 11 calculates a fairness index value based on the subjective best arm probability difference and the target value (step S125).

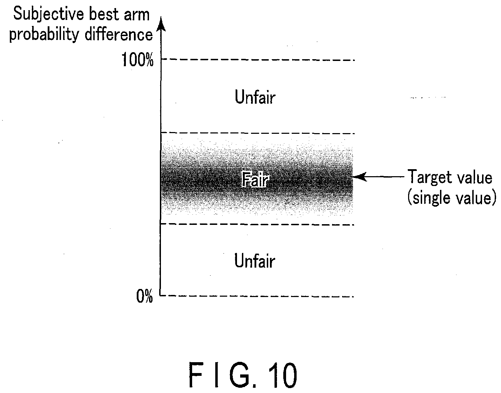

FIG. 10 is a diagram showing a fairness index value in the case of a target value being a single value. As shown in FIG. 10, in Example 2, a target value is set to a single value of the subjective best arm probability difference. The target value is set to the fairness index value of "1" indicative of fairness. The fairness index value is set continuously or discretely in such a manner as to approach "0", indicative of unfairness as the difference from the target value increases.

The processing circuitry 11 according to Example 2 calculates a target value based on "an effect evaluation value regarding a past medical decision of a subject target" and/or "an effect evaluation value for a medical decision selected by a subject other than one or more subject targets. That is, a target value is variable in response to another subject's selection of a medical decision and is also variable even with the same subject in response to medical decisions made right up to the present.

As an example, the processing circuitry 11 updates a target value $\Delta p_{target}$ based on an index moving average of a first derivation of an average of subjective best arm probability differences among subjects according to the following expression (3). $\Delta p_e$ presents the average value of the subjective best arm probability differences of a subject e, and $\Delta p_{e-1}$ presents the average value of the subjective best arm probability differences of a subject e-1. A subject e-1 is a person to whom a medical decision was assigned immediately before a subject e. In the expression (3), $\beta$ represents a hyperparameter for calculating momentum. According to the expression (3), the target value $\Delta p_{target}$ of the subject target "target" is updated based on the average value $\Delta p_e$ of the subjective best arm probability differences of the subject e and the average value $\Delta p_{e-1}$ of the average subjective best arm probability differences of the subject e-1.

$$\Delta p_{target} \leftarrow \mu + \nu \qquad (3)$$
$$\nu \leftarrow \beta\nu + (1-\beta)(\overline{\Delta p_e} - \overline{\Delta p_{e-1}})$$
$$\mu \leftarrow \beta\mu + (1-\beta)\overline{\Delta p_e}$$

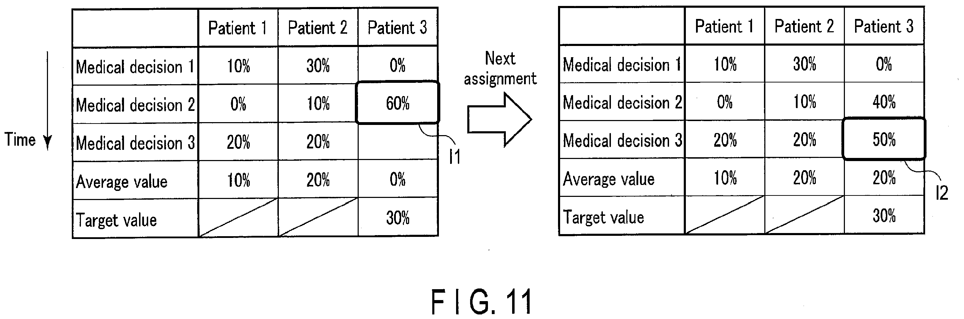

FIG. 11 is a diagram showing changes in assignment policy for medical decisions. In FIG. 11, a patient 3 corresponds to the subject target "target" as an assignment target to be assigned with a medical decision. FIG. 11 shows a phase of determining assignment of a second medical decision I1. As shown in the left portion of FIG. 11, with respect to a patient 1, the subjective best arm probability difference of the first medical decision is "10%", the subjective best arm probability difference of the second medical decision is "0%", and the subjective best arm probability difference of the third medical decision is "20%", so that the average value $\Delta p_{e-1}$ of the subjective best arm probability differences of the patient 1 is "10%". With respect to a patient 2, the subjective best arm probability difference of the first medical decision is "30%", the subjective best arm probability difference of the second medical decision is "10%", and the subjective best arm probability difference of the third medical decision is "20%", so that the average value $\Delta p_e$ of the subjective best arm probability differences of the patient 2 is "20%". With respect to a patient 3, the subjective best arm probability difference of the first medical decision is "0%", and the average value of the subjective best arm probability differences is "0%". In this case, according to the above expression (3), supposing that the hyperparameter $\beta=0$, the target value $\Delta p_{target}$ of a patient is "30%". This means that a model aims to select, as the second medical decision I1 of the patient 3, a medical decision with the subjective best arm probability difference of "60%". However, a medical decision with the subjective best arm probability difference of "60%" may not exist in some cases, and even in the presence of such a medical decision, the patient B does not always select this medical decision in reality.

As shown in the right portion of FIG. 11, assume that the patient 3 selects, as the second medical decision, a medical decision with the subjective best arm probability difference of "40%". In this case, an average value of the subjective best arm probability differences of the patient 3 is (0%+40%)/2=20%. As the third medical decision I2 of the patient 3, the model aims to select a medical decision with the subjective best arm probability difference of "50%" in such a manner that the average value of the subjective best arm probability differences of the third medical decision matches the target value. As described above, the sequential decision making sets a single target value. This causes this time's medical decision assignment policy to be changed in response to the subjective best arm probability difference of the past medical decision selected by a subject himself or herself. This realizes an adaptive assignment of a medical decision with respect to the latest selection by a subject himself or herself.

As in the examples described above, a target value is not only set based on the average value of the subjective best arm probability differences of other subjects but also set based only on the average value of the subjective best arm probability differences of a subject concerned or based on both of the average value of the subject best arm probability differences of the patient concerned and the average value of the subject best arm probability differences of other subjects.

After step S125, by implementing the assignment determination function 118, the processing circuitry 11 sets a medical decision to be assigned as an assignment subject by selecting it from assignment candidates (step S126).

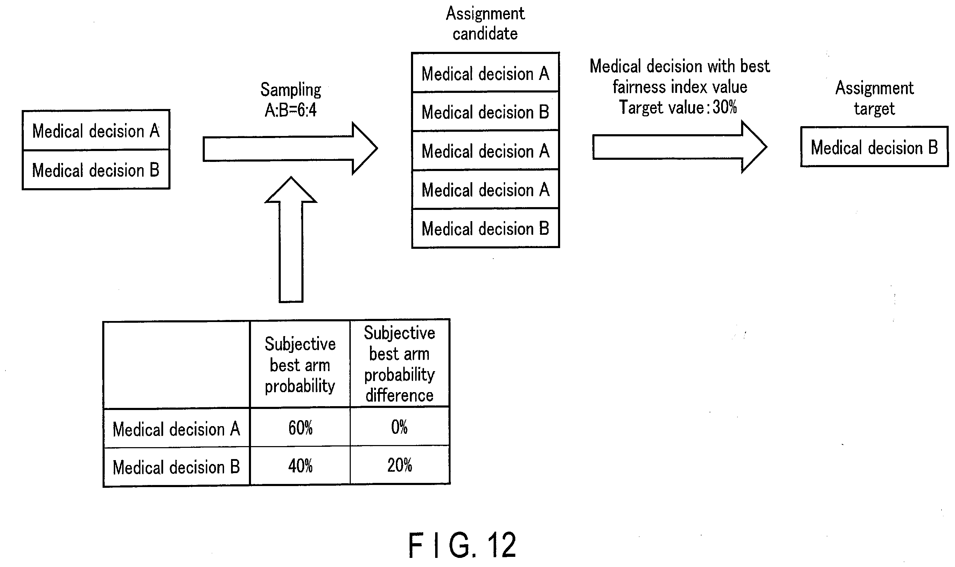

FIG. 12 is a diagram showing a specific example of setting processing of an assignment subject. As shown in FIG. 12, the medical decision A exhibits the subjective best arm probability of "60%" and the subjective best arm probability difference of "0%", while the medical decision B exhibits the subjective best arm probability of "40%" and the subjective best arm probability difference of 20%. In step S122, the medical decision A and the medical decision B are stochastically sampled by the number of random numbers with an assignment probability (sampling probability) according to the subjective best arm probability. In the example shown in FIG. 12, in the presence of five random numbers, the medical decision A and medical decision B are sampled five times at the ratio of 6:4, so that five allocation candidates are obtained. In step S126, an assignment candidate with the best fairness index value is selected from assignment candidates and is set to the assignment subject.

Herein, if the target average value of the subjective best arm probability differences is "10%" and the average value of the subjective best arm probability differences in the past two selections is "0%", the medical decision with the subjective best arm probability difference of "30%" is ideally set to the assignment subject in order for the average value of the subjective best arm probability differences in three selections including the past two selections and this time's selection to approximate the target average value of "10%". In this case, the target value is set to "30%". The processing circuitry 11 calculates the fairness index value based on the difference between the target value and the subjective best arm probability difference for each assignment candidate. The fairness index value ranges, for example, from the maximum value of "1" indicative of unfairness to the minimum value of "0" indicative of fairness, and is set such that the fairness index value approximates the minimum value "0" as the difference value decreases. The assignment candidate A exhibits the subjective best arm probability difference of "0%" and thus the fairness index value of "0.3". The assignment candidate B exhibits the subjective best arm probability difference of "20%" and thus the fairness index value of "0.1". The assignment candidate B is smaller in fairness index value than the assignment candidate A, so that the assignment candidate B is set to an assignment subject.

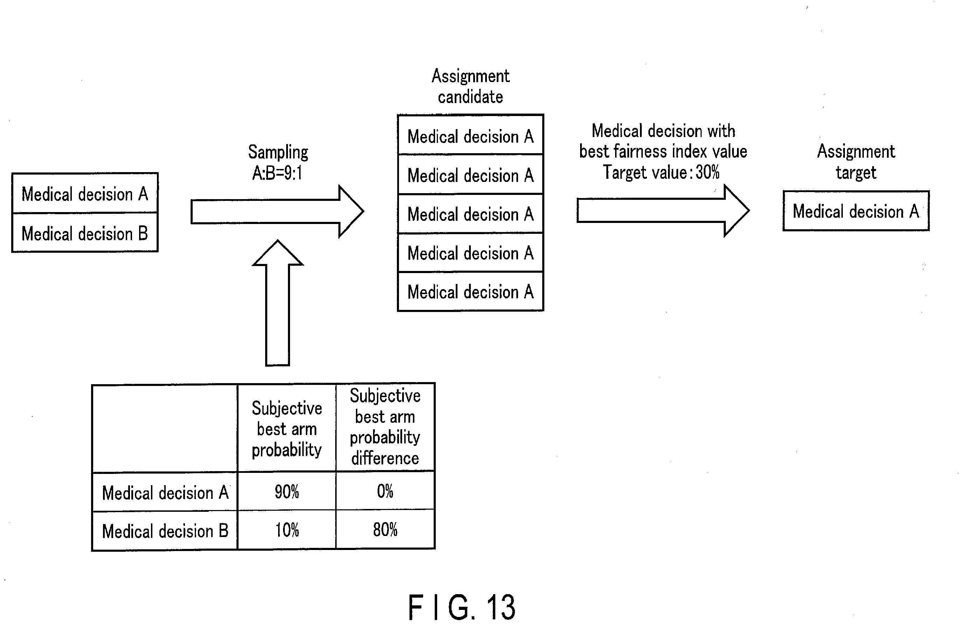

FIG. 13 is a diagram showing another specific example of setting processing of an assignment subject. As shown in FIG. 13, the medical decision A exhibits the subjective best arm probability of "90%" and the subjective best arm probability difference of "0%", while the medical decision B exhibits the subjective best arm probability of "10%" and the subjective best arm probability difference of 80%. In the example shown in FIG. 13, the medical decision A and medical decision B are sampled five times at the ratio of 9:1, so that five allocation candidates are obtained. If the target average value of the subjective best arm probability differences is "40%" and the average value of the subjective best arm probability differences in the past two selections is "0%", the medical decision with the subjective best arm probability difference of "120%" is ideally set to the assignment subject in order for the average value of the subjective best arm probability differences in three selections to approximate the target average value of "40%". Herein, of the medical decisions A and B, the subjective best arm probability difference of the medical decision B is approximate to "120%".

However, the processing circuitry 11 sets the medical decision A to the assignment subject because the medical decision B is not sampled as the assignment candidate.

The assignment processing according to Example 2 is thus completed.

As described above, the processing circuitry 11 according to Example 2 determines a plurality of random numbers from among a plurality of medical decisions in accordance with a predetermined probability distribution, and based on the determined random numbers, stochastically determines a plurality of assignment candidates in accordance with a predetermined policy. The processing circuitry 11 determines a medical decision to be assigned as an assignment subject, based on a comparison of fairness index values between the assignment candidates. More specifically, the processing circuitry 11 sets the allocation candidate with the best fairness index value from among the plurality of allocation candidates as the allocation target. The method according to Example 2 samples a plurality of assignment candidates while setting a base to a bandit algorithm that stochastically determines a medical decision, thereby being able to increase the probability of selecting a medical decision approximate to a target value of the subjective best arm probability difference. Furthermore, the method according to Example 2 calculates a target value based on "a past effect evaluation value for a subject target" and/or "an effect evaluation value for a medical decision selected by a subject other than one or more subject targets", so that the target value is applicable to sequential decision making.

Other examples

Examples 1 and 2 described above are examples of the medical information processing according to the present embodiment, and the steps of the medical information processing according to the present embodiment are not limited thereto. The medical information processing apparatus according to the present embodiment includes an apparatus that takes any step as long as it determines a medical decision to be assigned as an assignment subject, based on an effect evaluation value and a fairness index value.

In some of the examples described above, the processing circuitry 11 is configured to calculate the effect evaluation value by implementing the effect evaluation value acquisition function 116. However, the present embodiment is not limited to this. For example, the effect evaluation value may be calculated by a computer separate from the medical information processing apparatus 1, and the processing circuitry 11 may acquire the effect evaluation value from the computer. Alternatively, the processing circuitry 11 may acquire the effect evaluation value from a database that stores and manages the effect evaluation values.

In some of the examples described above, the processing circuitry 11 is configured to calculate the fairness index value by implementing the fairness index value acquisition function 117. However, the present embodiment is not limited to this. For example, the fairness index value may be calculated by a computer separate from the medical information processing apparatus 1, and the processing circuitry 11 may acquire the fairness index value from the computer. Alternatively, the processing circuitry 11 may acquire the fairness index value from a database that stores and manages the fairness index values.

In the case where an effect depends not only on a medical decision but also on a subject's feature amount such as an attribute, a condition, etc., an effect evaluation value may be calculated separately for each feature amount or may be calculated by a model expression presenting a relationship between a feature amount and an effect. Based on the above, the present embodiment is applicable to contextual bandit and sequential decision making. For example, use of a technique such as PSRL, BDQN, soft actor critic (SAC), etc., enables a subjective best arm probability to be calculated even with an estimated value of an effect.

Remarks

According to some of the examples described above, the medical information processing apparatus 1 according to the present embodiment includes the processing circuitry 11. The processing circuitry 11 acquires an effect evaluation value regarding a plurality of medical decisions, acquires a fairness index value regarding the plurality of medical decisions, and determines a medical decision to be assigned to a subject target based on the effect evaluation value and the fairness index value.

With the configuration described above, a medical decision to be assigned as an assignment subject can be determined by using not only the effect evaluation value but also the fairness index value. This allows a plurality of subjects to fairly select an exploratory action.

According to at least one embodiment described above, unfairness among subjects in assignment of medical decisions can be reduced while maintaining the magnitude of benefits for subjects in general.

The term "processor" used herein refers to, for example, a CPU or a GPU, or various types of circuitry, such as an application-specific integrated circuit (ASIC), a programmable logic device (e.g., a simple programmable logic device (SPLD), a complex programmable logic device (CPLD), or a field programmable gate array (FPGA)), and so on. The processor implements functions by reading and executing a program stored in a memory circuit. The programs may be incorporated directly in circuits of the processor, instead of being stored in the storage circuitry. According to such architecture, the processor reads the programs incorporated in its circuits and executes them to realize the functions. As another option, functions corresponding to the programs may be realized by a combination of logic circuits, instead of having the programs executed. The embodiments, etc., described herein do not limit each processor to a single circuitry-type processor. Multiple independent circuits may be combined and integrated as one processor to realize the intended functions. Furthermore, multiple components or features as given in FIG. 1 may be integrated as one processor to realize the respective functions.

While certain embodiments have been described, these embodiments have been presented by way of example only, and are not intended to limit the scope of the inventions. Indeed, the novel embodiments described herein may be embodied in a variety of other forms. Furthermore, various omissions, substitutions, and changes in the form of the embodiments may be made without departing from the spirit of the inventions. The accompanying claims and their equivalents are intended to cover such forms or modifications as would fall within the scope and spirit of the inventions.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

Regarding the foregoing embodiments, the appendage of the following is disclosed as one aspect and set of selective features of the invention.

**Claims**

1.  A medical information processing apparatus comprising processing circuitry configured to:

    acquire an effect evaluation value regarding a plurality of medical decisions;
    acquire a fairness index value regarding the medical decisions; and
    determine a medical decision to be assigned to a subject target based on the effect evaluation value and the fairness index value.

2.  The medical information processing apparatus according to claim 1, wherein

    the fairness index value is a value based on the effect evaluation value, and
    the processing circuitry calculates the fairness index value based on the effect evaluation value.

3.  The medical information processing apparatus according to claim 2, wherein

    the fairness index value is a value based on the effect evaluation value and a target value with respect to the effect evaluation value set for a respective one of subjects including the subject target, and
    the processing circuitry is configured to calculate the fairness index value based on the effect evaluation value and the target value.

4.  The medical information processing apparatus according to claim 3, wherein the processing circuitry sets the target value based on an effect evaluation value regarding a past medical decision for the subject target.

5.  The medical information processing apparatus according to claim 3, wherein the processing circuitry is configured to set the target value based on an effect evaluation value regarding a medical decision selected by a subject other than the target subject.

6.  The medical information processing apparatus according to claim 1, wherein the processing circuitry is configured to:

    determine an assignment candidate from among the plurality of medical decisions based on the effect evaluation value for a respective one of the medical decisions; and
    determine the medical decision to be assigned based on the fairness index value of the assignment candidate.

7.  The medical information processing apparatus according to claim 6, wherein in a case where a fairness index value regarding the assignment candidate satisfies a predetermined condition, the processing circuitry determines that the assignment candidate is the medical decision to be assigned.

8. The medical information processing apparatus according to claim 1, wherein the processing circuitry is configured to:

   stochastically sample a plurality of assignment candidates from the plurality of medical decisions based on a plurality of random numbers in accordance with a predetermined policy; and
   determine the medical decision to be assigned based on the fairness index value of the plurality of assignment candidates.

9. The medical information processing apparatus according to claim 8, wherein the processing circuitry determines the medical decision to be assigned based on a comparison of fairness index values between the assignment candidates.

10. The medical information processing apparatus according to claim 8, wherein the effect evaluation value is based on a posterior distribution of an effect with respect to a respective one of the medical decisions.

11. The medical information processing apparatus according to claim 8, wherein the effect evaluation value is calculated based on an expression:

$$p_i = \int_{-\infty}^{\infty} q_i(\alpha|D_i) \prod_{i \neq j} \left( \int_{-\infty}^{\alpha} q_j(\beta|D_j)\, d\beta \right) d\alpha$$

wherein $q_i(\alpha|D_i)$ is a first posterior probability density function of an effect of medical decision i under a condition in which effect observation value $D_i$ is obtained, and $\alpha$ is a parameter of the first posterior probability density function, and wherein $q_i(\beta|D_i)$ is a second posterior probability density function of an effect of medical decision j under a condition in which effect observation value $D_j$ is obtained, and $\beta$ is a parameter of the second posterior probability density function.

12. A medical information processing method comprising:

   acquiring an effect evaluation value regarding a plurality of medical decisions;
   acquiring a fairness index value regarding the medical decisions; and
   determining a medical decision to be assigned to a subject target based on the effect evaluation value and the fairness index value.

13. A non-transitory computer-readable storage media recording a medical information processing program for causing a computer to implement functions of:

   acquiring an effect evaluation value regarding a plurality of medical decisions;
   acquiring a fairness index value regarding the medical decisions; and
   determining a medical decision to be assigned to a subject target based on the effect evaluation value and the fairness index value.

1

Memory 12

Input device 13

Communication device 14

Display device 15

Processing circuitry 11

Assignment function 111

Effect evaluation value acquisition function 116

Fairness index value acquisition function 117

Assignment determination function 118

Observation function 112

Accumulation function 113

Updating function 114

Display control function 115

F I G. 1

```
        ┌─────────────┐
        │    Start    │
        └─────────────┘
               │
               ▼
 ┌───────────────────────────────────────────────────┐
 │  ┌─────────────────────────────────────────┐       │
 │  │  Calculate effect evaluation value       │ ～S101 │
 │  │  using model                             │       │
 │  └─────────────────────────────────────────┘       │
 │               │                                     │
 │               ▼                                     │
 │  ┌─────────────────────────────────────────┐       │     ～S1
 │  │  Calculate fairness index value          │ ～S102 │
 │  └─────────────────────────────────────────┘       │
 │               │                                     │
 │               ▼                                     │
 │  ┌─────────────────────────────────────────┐       │
 │  │  Determine medical decision to be        │       │
 │  │  assigned to subject based on effect     │ ～S103 │
 │  │  evaluation value and fairness index     │       │
 │  │  value                                   │       │
 │  └─────────────────────────────────────────┘       │
 └───────────────────────────────────────────────────┘
               │
               ▼
 ┌───────────────────────────────────────────────────┐
 │       Observe effect produced on subject           │ ～S2
 └───────────────────────────────────────────────────┘
               │
               ▼
 ┌───────────────────────────────────────────────────┐
 │            Accumulate observation data             │ ～S3
 └───────────────────────────────────────────────────┘
               │
               ▼
 ┌───────────────────────────────────────────────────┐
 │            Update parameter of model               │ ～S4
 └───────────────────────────────────────────────────┘
               │
               ▼
        ┌─────────────┐
        │     End     │
        └─────────────┘
```

FIG. 2

F I G. 3

Start

Calculate subjective best arm probability
for each medical decision — S111

Determine assignment candidate based
on subjective best arm probability
in accordance with bandit algorithm — S112

Calculate subjective best arm
probability difference for assignment candidate — S113

Set target value of subjective best arm
probability difference — S114

Calculate fairness index value
"0" (fair) or "1" (unfair)
based on subjective best arm
probability difference and target value — S115

S116
Fairness index value=0? —— NO ——

YES — S117

Set assignment candidate to medical
decision of assignment subject

S118
Set another medical decision to medical
decision serving as assignment target

End

F I G. 4

Medical decision A
Medical decision B
Medical decision C
Medical decision D
Medical decision E
Medical decision F

Bandit algorithm (Single random number)

Assignment candidate

Medical decision C

Fairness index value=0(fair)?

○

Adoption

Assignment target

Medical decision C

✕

Elimination

Assignment target

Medical decision F

Select from medical decisions other than assignment candidates

F I G. 5

EP 4 160 611 A1

Subjective best arm probability

10%                    90%

~a1                    ~a2

80%                    0%          → Treatment effect

Subjective best arm probability difference

# F I G. 6

Subjective best arm
probability difference

100% ┝ ─ ─ ─ ─ ─ ─ ─ ─ ─ ─ ─ ─ ┐

Unfair

Threshold value ┝ ─ ─ ─ ─ ─ ─ ─ ─ ─ ─ ─ ┐

Fair          │ Target value (range)

0% └ ─ ─ ─ ─ ─ ─ ─ ─ ─ ─ ─ ─ ┘

# F I G. 7

```
                    ┌──────────────┐
                    │    Start     │
                    └──────┬───────┘
                           │
                           ▼
        ┌──────────────────────────────────────┐
        │   Calculate subjective best arm       │
        │   probability for each medical decision│ ~S121
        └──────────────────┬───────────────────┘
                           │
                           ▼
        ┌──────────────────────────────────────┐
        │   Determine multiple assignment       │
        │   candidates based on subjective best │ ~S122
        │   arm probability in accordance with  │
        │   bandit algorithm                    │
        └──────────────────┬───────────────────┘
                           │
                           ▼
        ┌──────────────────────────────────────┐
        │   Calculate subjective best arm       │
        │   probability difference for each     │ ~S123
        │   assignment candidate                │
        └──────────────────┬───────────────────┘
                           │
                           ▼
        ┌──────────────────────────────────────┐
        │   Calculate target value of subjective │
        │   best arm probability difference     │ ~S124
        └──────────────────┬───────────────────┘
                           │
                           ▼
        ┌──────────────────────────────────────┐
        │   Calculate fairness index value based │
        │   on subjective best arm              │ ~S125
        │   probability difference and target value│
        └──────────────────┬───────────────────┘
                           │
                           ▼
        ┌──────────────────────────────────────┐
        │   Set medical decision serving as     │
        │   assignment candidate from assignment│ ~S126
        │   candidates based on fairness index  │
        │   value                               │
        └──────────────────┬───────────────────┘
                           │
                           ▼
                    ┌──────────────┐
                    │     End      │
                    └──────────────┘
```

# F I G. 8

Assignment
candidate

| Medical decision A |
| Medical decision B |

Bandit algorithm
(Multiple random numbers)

→

| Medical decision A |
| Medical decision B |
| Medical decision A |
| Medical decision A |
| Medical decision B |

Medical decision with
best fairness index value

→

Assignment
target

| Medical decision B |

History of medical decisions
regarding subject target
and another subject

# FIG. 9

EP 4 160 611 A1

F I G. 10

Time ↓

| | Patient 1 | Patient 2 | Patient 3 |
|---|---|---|---|
| Medical decision 1 | 10% | 30% | 0% |
| Medical decision 2 | 0% | 10% | 60% |
| Medical decision 3 | 20% | 20% | |
| Average value | 10% | 20% | 0% |
| Target value | | | 30% |

I1

Next assignment ⇒

| | Patient 1 | Patient 2 | Patient 3 |
|---|---|---|---|
| Medical decision 1 | 10% | 30% | 0% |
| Medical decision 2 | 0% | 10% | 40% |
| Medical decision 3 | 20% | 20% | 50% |
| Average value | 10% | 20% | 20% |
| Target value | | | 30% |

I2

F I G. 11

Assignment
candidate

| Medical decision A |
|---|
| Medical decision B |

Sampling
A:B=6:4

| Medical decision A |
|---|
| Medical decision B |
| Medical decision A |
| Medical decision A |
| Medical decision B |

Medical decision with best
fairness index value
Target value:30%

Assignment
target

| Medical decision B |
|---|

| | Subjective best arm probability | Subjective best arm probability difference |
|---|---|---|
| Medical decision A | 60% | 0% |
| Medical decision B | 40% | 20% |

FIG. 12

FIG. 13

**EUROPEAN SEARCH REPORT**

Application Number

EP 22 19 8884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PRESS WILLIAM H.: "Bandit solutions provide unified ethical models for randomized clinical trials and comparative effectiveness research", PROCEEDINGS OF THE NATIONAL ACADEMY OF SCIENCES, vol. 106, no. 52, 29 December 2009 (2009-12-29), pages 22387-22392, XP093018442, ISSN: 0027-8424, DOI: 10.1073/pnas.0912378106 * p. 22387, title 22387, right column, par. 1, lines 3 - 6 p. 22387, right column, par. 3, lines 1 - 9 p. 22387, right column, Methods, par. 1 -3 p. 22387, eq. [1] p. 22388, eq. [7] p. 22388, left column, par. 7, lines 7f p. 22389, left column, par. 2 p. 22389, right column, par. 2, lines 3-6 p.22389 eq. [12] and eq. [13] appendix, p. 3, (7) * & Press William H: "Supporting Information for "Bandit solutions provide unified ethical models for randomized clinical trials and comparative effectiveness research" State Variables and Successor Points", Proc. Natl. Acad. Sci. U.S.A, 29 December 2009 (2009-12-29), pages 1-5, XP093018443, Retrieved from the Internet: URL:https://www.pnas.org/doi/10.1073/pnas.0912378106#supplementary-materials [retrieved on 2023-01-27] ----- -/-- | 1-13 | INV. G16H40/20 |
| | | | TECHNICAL FIELDS SEARCHED (IPC) |
| | | | G16H |

The present search report has been drawn up for all claims

| Place of search | Date of completion of the search | Examiner |
|---|---|---|
| Munich | 30 January 2023 | Mühlbauer, Max |

Europäisches
Patentamt

European
Patent Office

Office européen
des brevets

## EUROPEAN SEARCH REPORT

Application Number

EP 22 19 8884

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2008/301077 A1 (FUNG GLENN [US] ET AL) 4 December 2008 (2008-12-04) * the whole document * ----- | 1-13 | |
| A | YANGYI LU ET AL: "Bandit Algorithms for Precision Medicine", ARXIV.ORG, CORNELL UNIVERSITY LIBRARY, 201 OLIN LIBRARY CORNELL UNIVERSITY ITHACA, NY 14853, 10 August 2021 (2021-08-10), XP091031176, * the whole document * ----- | 1-13 | |

TECHNICAL FIELDS SEARCHED (IPC)

The present search report has been drawn up for all claims

| Place of search | Date of completion of the search | Examiner |
|---|---|---|
| Munich | 30 January 2023 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 8884

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2023

| Patent document cited in search report | Publication date | Patent family member(s) | Publication date |
|---|---|---|---|
| US 2008301077 A1 | 04-12-2008 | US 2008301077 A1 | 04-12-2008 |
| | | WO 2008150514 A2 | 11-12-2008 |

EPO FORM P0459

For more details about this annex : see Official Journal of the European Patent Office, No. 12/82

**EP 4 160 611 A1**

**Patent documents cited in the description**

- JP 2021160732 A